# EUROPEAN PATENT SPECIFICATION

(11) **EP 2 512 387 B1**
(45) Date of publication and mention of the grant of the patent: **30.09.2015**
(21) Application number: 10822863.6
(22) Date of filing: 14.12.2010
(51) Int. Cl.: A61F 5/01

(54) **ORTHOPEDIC DEVICE**
ORTHOPÄDISCHE VORRICHTUNG
DISPOSITIF ORTHOPÉDIQUE

(30) Priority: 08.09.2010 US 877361; 06.05.2010 US 774882; 17.12.2009 US 640232
(43) Date of publication of application: 24.10.2012
(73) Proprietor: Össur HF, 110 Reykjavik (IS)
(72) Inventor: INGIMUNDARSON, Arni, Thor, Foothill Ranch, CA 92610 (US); EINARSSON, Palmi, Foothill Ranch, CA 92610 (US)
(74) Representative: Klunker . Schmitt-Nilson . Hirsch
(86) International application number: PCT/IB2010/003540
(87) International publication number: WO 2011/073803

(56) References cited:
- US-A1- 2006 135 903
- US-A1- 2007 083 136
- US-A1- 2007 185 425
- US-A1- 2007 225 824
- US-A1- 2009 099 562
- US-B1- 6 592 539
- US-B2- 7 161 056

## Description

### [1] BACKGROUND

One type of knee infirmity that many individuals are prone to having is compartmental osteoarthritis. Compartmental osteoarthritis may arise when there is a persistent uneven distribution of pressure in one of the medial and lateral compartments of the knee. Compartmental osteoarthritis can be caused by injury, obesity, misalignment of the knee, or simply due to aging of the knee.

A major problem resulting from osteoarthritis of the knee is that the smooth cartilage lining the inside of the knee wears away. This leads to a narrowing of the joint space with the development of cysts and erosions in the bone ends. Because of the narrowing of the joint, bone comes directly in contact with bone, and an uneven distribution of pressure develops across the knee which may result in the formation of bone spurs around the joint. All of these changes ultimately lead to increasing pain and stiffness of the joint.

While there are no cures to osteoarthritis, there are many treatments. Individuals who have a diagnosis of isolated medial compartmental osteoarthritis of the knee are confronted with a variety of treatment options such as medications, surgery, and nonsurgical interventions. Nonsurgical interventions include the use of canes, lateral shoe wedges, and knee bracing.

Knee bracing is useful to provide compartment pain relief by reducing the load on the compartment through the application of an opposing external valgus or varus moment about the knee joint. Unloading knee braces have been shown to significantly reduce osteoarthritis knee pain while improving knee function.

There are many known unloading knee braces. An example of a known brace is described in U.S. Patent 5,277,698. Typically, braces of this type are designed to apply a moment about the knee through two mechanisms. The first mechanism is through the angulations of hinge components which induce a bending moment at a hinge. The second mechanism is provided by a three-point bending system via a force strap that spirals around the knee and applies a force to a prescribed aspect of the knee.

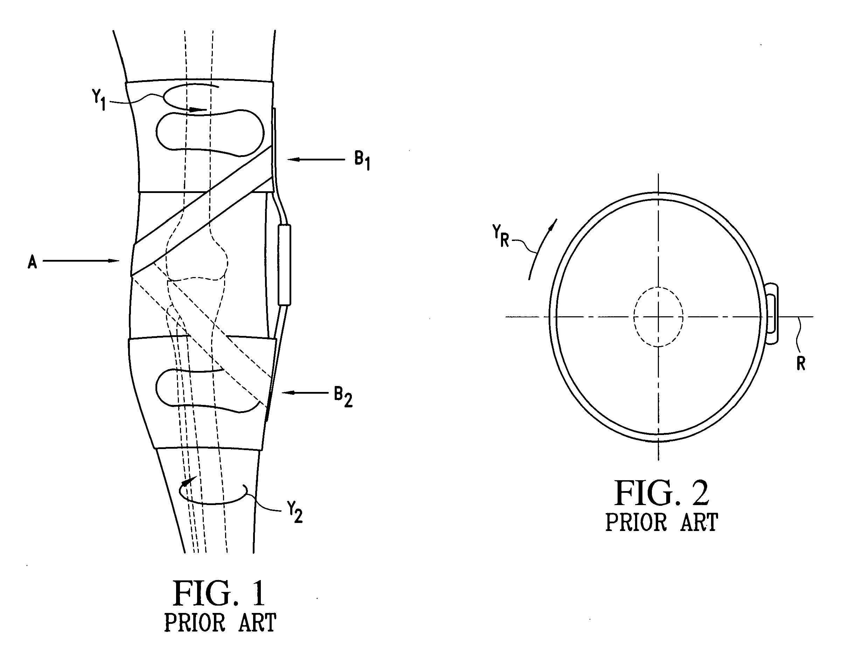

Figs. 1 and 2 exemplify the application of forces by the brace on a leg and over a knee joint according to U.S. Patent 5,277,698. The arrows B₁ and B₂ show lateral and force strap forces. The resulting moments in the leg due to lateral forces are shown by arrows Y₁ and Y₂. The principal force A is that applied immediately adjacent that compartment of the knee having osteoarthritis. Fig. 2 shows R as the normal axis of rotation of the knee. The resultant moment Y_{R} is a single rotational moment.

It has been found that as the force strap is increased in tension, the hinge valgus producing moment decreases. Therefore, the force strap and the hinge are found not to be adequately working in harmony. More specifically, it was discovered that the hinge produces about 20% of the total valgus moment in this brace. It is believed that since the hinge is aligned close to the knee, the strap urges the knee against the hinge. Moreover, the rigidity of this type of hinge limits the displacement of the hinge relative to the knee.

In a conventional brace having a hinge, a clearance is provided between the hinge and the knee to allow for movement of the knee towards the hinge. This results in a bulky brace since a large hinge is required which may extend at least an inch away from the knee.

It has been determined that if more unloading of the knee is required by the brace than is obtained from normal strap tension, and if the force strap is further tightened, the knee is drawn towards the hinge and might strike the hinge. This results in the hinge applying forces to the knee that counteract the force applied by the force strap. In turn, the additional tightening of the force strap is mitigated or negated by the force exerted onto the knee from the hinge.

For example, a study was conducted on a patient wearing a conventional knee brace having a force strap. In normal strap tension, the force strap component unloaded 5.8 Nm of the knee and the hinge unloaded about 2.2 Nm. By increasing the force strap tension, the unloading of the force strap resulted in 11.6 Nm, but the hinge resulted in unloading -2.4 Nm since the hinge was pressed against the knee.

Another known brace is described in US 2007/0185425 A1. US 2007/0185425 A1 discloses an orthotic or prosthetic spacer element including a ventilated and compressible core having first and second opposed surfaces, and a discrete and continuous web-like silicone-based frictional layer secured to the core first surface. The frictional layer and the core first surface have a plurality of coinciding apertures formed in a pattern. The spacer element may include a foam layer secured to the core second surface such that the foam layer and the core have different rigidities.

As will be more fully evident in the ensuing discussion, the embodiments described herein are provided to overcome the deficiencies of prior art unloading braces by including arrangements that provide maximum unloading of the knee brace, while removing the mitigating effects of the hinges in known knee braces. Moreover, the embodiments of the invention are arranged for treating compartmental osteoarthritis, and have improved mechanical properties that remove undesirable rotational forces incurred by the brace and provide a more effective mechanism for generating a valgus or varus moment at the knee.

While known knee braces are successful at reducing pain at or stabilizing a knee joint, many users find these braces to be bulky, difficult to don, complicated to configure, and uncomfortable to wear. For these reasons, the embodiments described herein have streamlined features capable of providing relief for medial or lateral compartmental osteoarthritis, or functional stability of the knee without the attendant drawbacks of known unloading knee braces.

### SUMMARY

Embodiments of the present invention are described in connection to an improved orthopedic device or knee brace and knee bracing method that serve to reduce the effects of either medial compartmental or lateral compartmental osteoarthritis. Embodiments of the knee brace and variations of the knee bracing method reduce the effects of compartmental osteoarthritis by applying multiple forces to the knee on the side remote from the compartment having osteoarthritis while providing forces on the side of the compartment to maintain the brace securely on a leg while minimizing rotational forces. The embodiments of the features described herein are not limited to usage in a knee brace, and may be extended to a variety of orthopedic and prosthetic applications.

According to one embodiment, the knee brace is provided with at least one breathable spacer element having an inner surface connected to an inner facing surface of at least one of the proximal and distal members. The spacer element defines an outer surface opposing the inner surface and includes a frictional feature or layer.

While described herein in the exemplary embodiment of a knee brace, the spacer element described herein may be used in a variety of prosthetic or orthotic applications. In particular, the spacer element may be provided without any relationship to a particular prosthetic or orthotic device, and used in a variety of applications where frictional control, breathability, compression or padding is required or desirable.

In one example of the spacer element, both the spacer element and the frictional feature are perforated. According to this example, the spacer element is a textile having a surface with a plurality of apertures upon which a discrete and continuous web-like silicone frictional layer is secured. This yields a breathable and compressible spacer element that provides frictional resistance to forces exerted thereon.

For example, this spacer element may be used in combination with the knee brace embodiments described herein so as to prevent rotation of the knee brace on the leg of the wearer. Moreover, by providing the spacer element on both proximal and distal portions of the knee brace prevents the proximal and distal portions of the knee brace from drawing closer to one another due to the forces applied by the force straps.

In another example of the spacer element, a foam (or similar compressible material) layer is secured to the core along a surface opposite to the surface with the frictional layer. The foam layer preferably has a different rigidity than a rigidity of the textile core. In variations of this example, additional layers of foam (or similar compressible materials) may be used having different rigidities.

In yet another example of the spacer element, a hook-receivable material may be secured to the core on a side opposite to the side with the frictional layer.

In variations of the frictional layer, the frictional layer includes a plurality of apertures that correspond to apertures of the core. In an alternative variation, the frictional layer includes a plurality of apertures that are independent of apertures defined by the core. In such a variation, the frictional layer does not prevent breathability of the core.

In yet another variation of the frictional layer, the frictional layer may include areas having different thicknesses. The different thicknesses may be formed by molding, laminating or coating the frictional layer so as to form different thickness regions, or alternatively, the frictional layer may comprise different layers located at specific areas to obtain the different thicknesses. Furthermore, the frictional layer may include areas having different hardness properties relative to other areas.

In combination with the force straps and spacer elements of the knee brace described herein, one can achieve more unloading forces than with one force strap without increasing the pressure applied to the knee. This is due to the total unloading moment that is doubled with two force straps; the same amount of pressure is applied to the knee since there are two pressure points.

Second proximal and distal principal points of force are generated by the spacer elements secured to the frame members on a second side of the leg at locations above and below, respectively, the first proximal and distal principal points of force. The spacer elements maintain the knee brace on a leg and the frame members apart. The spacer elements may remove the need for a hinge as is used in the prior art braces.

In accordance with an embodiment of the invention, the spacer element includes a three-dimensional knit fabric layer having first and second fabric layers spaced by a ventilated and compressible core, the first fabric layer being a fabric mesh having a plurality of uniformly spaced apertures arranged in a pattern. A discrete and continuous web-like frictional layer is laminated onto the first fabric layer and has greater frictional properties than the fabric layer. The frictional layer has a plurality of apertures arranged in a pattern in direct correspondence to the pattern of the first fabric layer apertures. A compressible material layer has opposed first and second surfaces, wherein the first surface is secured to the second fabric layer. The compressible material layer has a first compression molded region with reduced thickness and increased density relative to a second region of the compressible material layer having a predetermined thickness.

Of course, the spacer elements are only exemplified herein in combination with a knee brace. However, the spacer elements may be used in a variety of orthopedic, prosthetic and other applications beyond just knee braces where frictional control, breathability, compression or padding is required or desirable. In addition, in combination with suitable securing elements, such as a strap, the spacer element may itself form an orthopedic or prosthetic support.

In another embodiment, which is not part of the invention, the spacer element has a first region including first and second fabric layers spaced by a first compressible padding layer, a second region including first and second fabric layers spaced by a second compressible padding layer, and a third region including the first and second fabric layers adjacent one another. The first region is spaced from the second region by the third region. The first padding layer has a greater density than the second padding layer such that the first padding layer is less firm than the second padding layer.

According to another embodiment, which is not part of the invention, the orthopedic device includes a rigid or semi-rigid lower frame element having opposed first and second sides. The frame element is adapted to extend about at least a portion of a limb. The orthopedic device includes a gastroc strap connected to the first and second sides of the frame element. The gastroc strap has a first end defined by a strap tab secured to the first side of the frame element, an elastic segment having a first end secured to the strap tab, and a spanning segment connected to a second end of the elastic segment and defining a second end of the gastroc strap. The spanning segment is adjustably secured to the second frame element side such that the spanning segment is adjustable in length relative to the second frame element side. A second side of the frame element defines a slot whereat the spanning segment secures and wraps over such that a first surface of the spanning segment secures to a second surface of the spanning segment.

In another embodiment, which is not part of the invention, a thigh strap is connected to the first and second sides of an upper frame element. The thigh strap has a first end defined by a strap tab secured to the first side of the frame element, a first flexible segment adjustably secured to the strap tab, an elastic segment having a first end releasably secured to the first flexible segment, and a second flexible segment releasably secured to a second end of the elastic segment. The second flexible segment is adjustably secured to the frame element second side such that the second flexible segment is adjustable in length relative to the frame element second side. The thigh strap is configured for extending laterally about and over the posterior thigh region of the upper leg.

A second side of the upper frame element defines a slot whereat the second flexible segment secures and wraps over such that a first surface of the second flexible segment secures to a second surface of the second flexible segment.

In another embodiment of the orthopedic device in the form of a knee brace, which is not part of the invention, includes a rigid or semi-rigid upper frame element adapted to extend about at least a portion of a limb of a wearer of the brace, and a rigid or semi-rigid lower frame element adapted to extend about at least a portion of a limb of a wearer of the brace. An upper pad is connected to the upper frame element and has an exterior surface adjacent to the upper frame element and an interior surface opposed from the exterior surface. The interior surface of the upper pad is smooth to human touch so as to provide low resistance to movement when pressed against skin of a wearer of the brace. Preferably, the interior surface of the upper pad is formed from a doeskin, microfiber or suede material.

The knee brace may also include a lower pad connected to the lower frame element and having an exterior surface adjacent to the lower frame element and an interior surface opposed from the exterior surface. The interior surface of the lower pad has a frictional coating providing substantially more resistance to movement than the interior surface of the upper pad when pressed against skin of a wearer of the brace.

This particular embodiment of the knee brace with the smooth interior surface on the upper pad is provided to allow the upper frame element to piston on the thigh of the wearer without irritating the skin of the wearer. The lower frame element, however, is held firmly against the shin or tibia portion of the wearer due to the substantially frictional interior surface of the lower pad. The upper pad therefore allows the upper frame element to accommodate movement of the thigh, whereas the lower pad holds the lower frame element (in combination with at least one strap) firmly against the shin of the leg such that the lower frame element does not shift upon movement of the leg.

Of course, other methods, embodiments, and variations thereof are described in greater detail in the following discussion.

### BRIEF DESCRIPTION OF THE DRAWINGS

These and other features, aspects, and advantages of the present invention will become better understood with regard to the following description, appended claims, and accompanying drawings where:
Fig. 1 is a schematic view of forces applied on a leg using a prior art knee brace.
Fig. 2 illustrates the rotational force applied on a leg by the prior art knee brace of Fig. 1.
Fig. 3 is a perspective view of a knee brace.
Fig. 4 is a front elevation view of the brace of Fig. 3.
Fig. 5 is a medial side elevation view of the brace of Fig. 3.
Fig. 6 is a lateral side elevation view of the brace of Fig. 3.
Fig. 7 is an exploded view of the brace of Fig. 3 without a sleeve.
Fig. 8 is a schematic view of forces applied on a leg using the brace shown in Fig. 3.
Fig. 9 illustrates the rotational force applied on a leg by the brace of Fig. 3.
Fig. 10 generally illustrates where the force is applied externally of the knee in the brace of Fig. 3.
Fig. 11 is a sectional view taken along line 11-11 of Fig. 10.
Fig. 12A is a sectional view taken along line 12A-12A of Fig. 7.
Fig. 12B is a schematic elevational view of the spacer element of Fig. 12A.
Fig. 12C is a sectional view of another variation of a spacer element.
Fig. 12D is a sectional view of another variation of a spacer element.
Fig. 12E is a sectional view of another variation of a spacer element.
Fig. 12F is a sectional view of another variation of a spacer element.
Fig. 12G is a plan view of an embodiment of a spacer element in accordance with the invention.
Fig. 12H is a sectional view taken along line 12H-12H of Fig. 12G.
Fig. 12I is a sectional view corresponding to detail 12I of Fig. 12H.
Fig. 12J is a sectional view taken along line 12J-12J of Fig. 12G
Fig. 12K is a sectional view taken along line 12K-12K of Fig. 12G.
Fig. 12L is a sectional view taken along line 12L-12L of Fig. 12G.
Fig. 12M is a plan view of another variation of a spacer element.
Fig. 12N is a plan view of another variation of a spacer element.
Fig. 12O is a sectional view taken along line 120-120 of Fig. 12N.
Fig. 12P is a plan view of another variation of the spacer element.
Fig. 13 is a perspective view of an orthopedic device.
Fig. 14 is a perspective view of another orthopedic device.
Fig. 15 is a perspective view of a first side of another orthopedic device.
Fig. 16 is a perspective view of a second side of the device of Fig. 14.
Fig. 17 is a plan view of a gastroc strap for use in the orthopedic device of Fig. 15.
Fig. 18 is a schematic view of the strap of Fig. 69 being placed over a leg of a wearer.
Fig. 19 is a plan view of a thigh strap for use in the orthopedic device of Fig. 14.
Fig. 20 is a schematic view of central strap pad for use in the orthopedic device of Fig. 14 prior to being placed over a leg of a wearer.
Fig. 21 is a schematic view of the central strap pad over the leg over a wearer.
Fig. 22 is a first schematic sectional view of the orthopedic device of Fig. 14 having location indicia.
Fig. 23 is a second schematic sectional view of the orthopedic device of Fig. 14 having location indicia.

### DETAILED DESCRIPTION OF VARIOUS EMBODIMENTS

### A. Overview

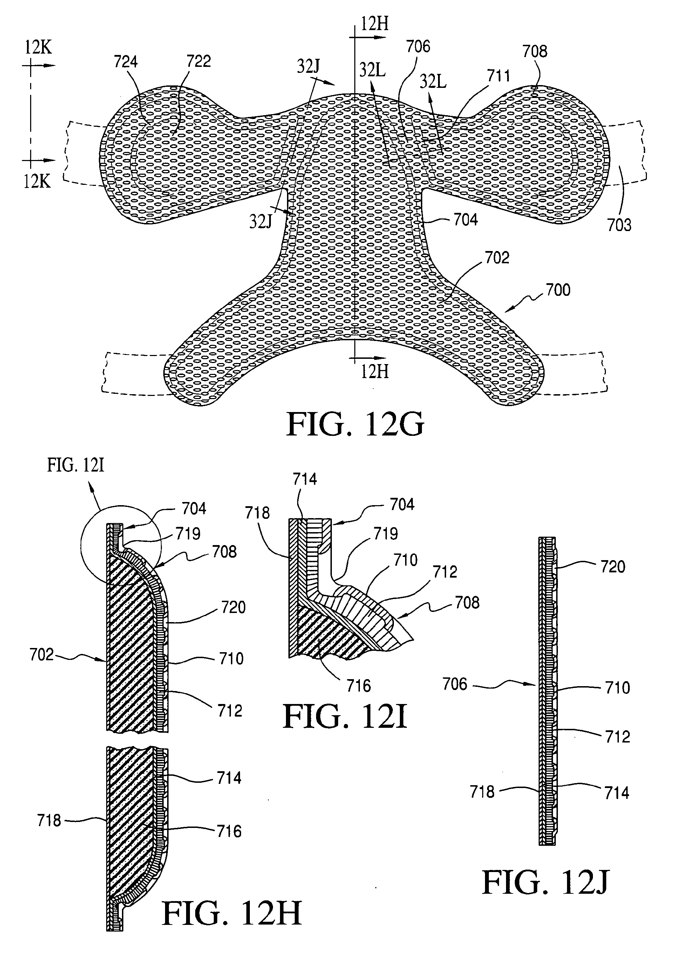

A better understanding of different embodiments of the invention may be had from the following description read in conjunction with the accompanying drawings in which like reference characters refer to like elements. The invention is shown in Figs. 12G to 12O.

While the disclosure is susceptible to various modifications and alternative constructions, certain illustrative embodiments are shown in the drawings and will be described below in detail. It should be understood, however, that there is no intention to limit the disclosure to the specific embodiments disclosed, but on the contrary, the intention is to cover all modifications, alternative constructions, combinations, and equivalents falling within the scope of the disclosure and defined by the appended claims.

It will be understood that, unless a term is expressly defined in this patent to possess a described meaning, there is no intent to limit the meaning of such term, either expressly or indirectly, beyond its plain or ordinary meaning.

### B. Environment and Context of Embodiments

Numerous embodiments of the invention are provided to reduce the effect of osteoarthritis in a knee joint, or stabilize a knee joint that has been weakened by injury or other infirmities. Embodiments of the invention may be configured to reduce or cure both medial and lateral knee joint infirmities.

Embodiments of the invention are particularly adapted for a human knee joint, and may be dimensioned to accommodate different types, shapes and sizes of human joints and appendages. In addition, embodiments may be modified to orient principal forces exerted by strap systems of the embodiments at any desirable location to treat knee infirmities.

For explanatory purposes, each knee brace embodiment described herein is divided into sections which are denoted by general anatomical terms for the human body. Each of these terms is used in reference to a human leg which is divided in similar sections with a proximal-distal plane generally extending along the meniscus of the knee between the femur and tibia.

The anatomical terms described herein are not intended to detract from the normal understanding of such terms as readily understood by one of ordinary skill in the art of orthotics.

### C. Various Embodiments of the Knee Brace

### i. Overview of Knee Brace Embodiments

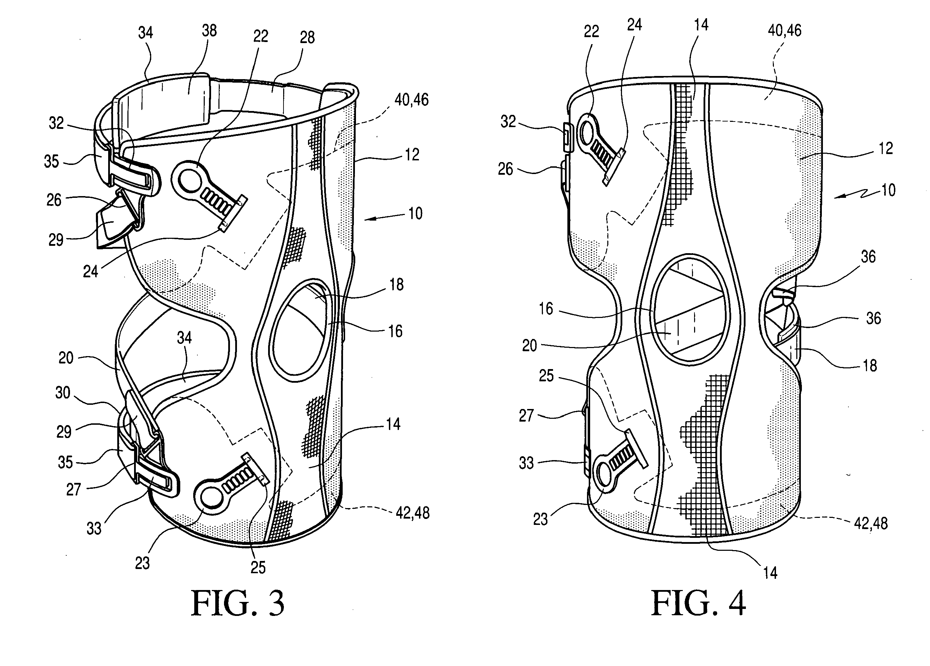

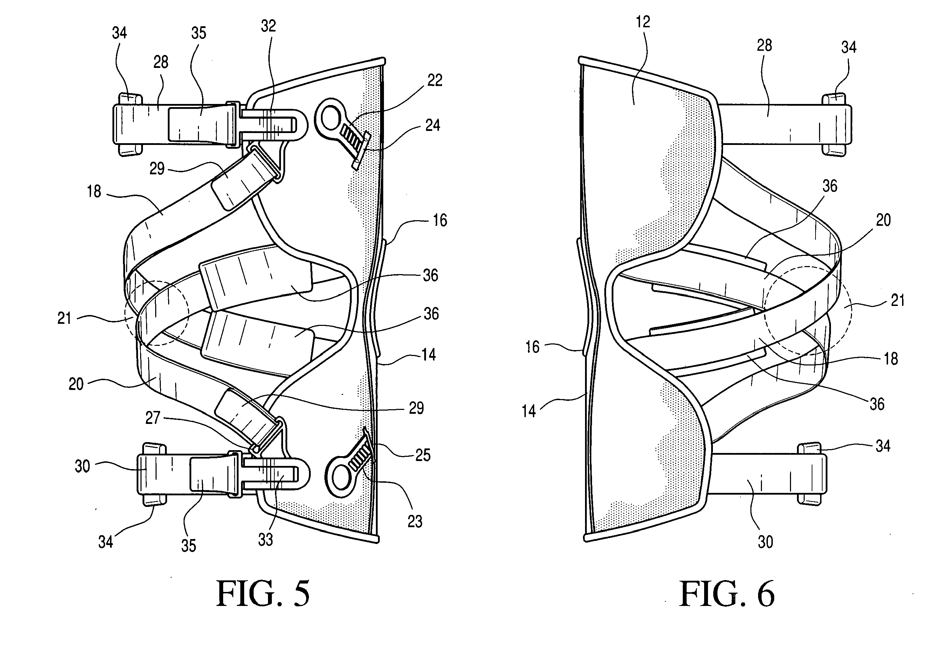

Referring to Figs. 3-6, a knee brace embodiment 10 is shown. While this knee brace is particularly shown and configured for treating lateral osteoarthritis of the knee, it is understood that the knee brace may be configured by reversing the features in order to treat medial osteoarthritis of the knee.

According to this embodiment, the brace 10 includes a sleeve 12 covering or upon which various components and assemblies are secured. As will be described below in reference to proximal and distal frame elements or shells 40, 42, these shells are connected to, inserted into, or secured against the sleeve to provide sufficient rigidity to the brace.

According to this embodiment, the sleeve 12 includes a breathable central strip portion 14 generally extending along the proximal-distal plane of the brace 10, and a center ring 16 located approximately about the center of the sleeve 12. The center ring 16 is preferably constructed from an stretchable material so as to provide sufficient flexure of the brace 10 about the center portion thereof, and is located so as to assist a user of the device in placing the center portion over the anterior knee. Moreover, the portion of the sleeve 12 corresponding to the proximal portion of the knee is left exposed in order prevent interference of extension and flexion of the knee.

First and second force straps 18, 20 are each secured at a first end to a corresponding tightening device 22, 23 that protrudes out of an opening 24, 25 of the sleeve 12. The second end of each of the force straps 18, 20 is secured to a corresponding bracket assembly 26, 27 also secured to the sleeve 12. The first and second force straps 18, 20 intersect at intersection point 21 that is located near or along the proximal-distal plane on the posterior, medial side of the brace 10.

Each of the force straps 18, 20 may include a cushion feature 36 that may be located near or at locations anterior or posterior of the intersection point 21. Moreover, the force straps 18, 20 preferably each have a length adjustment feature 29, such as a hook and loop fastener system, to enable adjustment of the length of such straps 18, 20.

In this embodiment, the force straps 18, 20 are substantially inelastic in order to apply a greater amount of pressure against the knee as opposed to what may occur if elastic straps are used. It has been found that force straps having substantially elastic properties do not effectively unload a knee. Instead, elastic force straps pull the knee into flexion such that when the leg is straightened, the force straps resist flexure of the knee. As a result, while tightening the force straps may indeed unload the knee, the knee is unable to undergo full extension due to the tendency of the knee to go into flexion. Unlike the elastic straps, substantially inelastic straps do not possess these drawbacks since they draw the knee towards a hinge and unload the knee while permitting both flexure and extension of the knee.

It should be understood, however, that embodiments of the knee brace are not limited to usage of substantially inelastic straps. To the contrary, straps of various degrees of elasticity may be employed with the various components in different the embodiments of the knee braces to suit various needs of an individual wearing the brace.

The first force strap 18 is secured to a lateral-proximal bracket assembly 26 and spirals along the posterior of the brace 10 towards the medial-distal side of the sleeve 12. The first force strap 18 then enters in the sleeve 12 and secures to a distal tightening device 23 generally located on the anterior-lateral, distal side of the sleeve 12.

The second force strap 20 is secured to a lateral-distal bracket assembly 27 and spirals around the posterior of the brace 10 towards the medial proximal side of the sleeve. The strap 20 then enters the sleeve 12 and secures to a proximal tightening device 22 generally located on the anterior-lateral proximal side of the sleeve 10. As will be described in the ensuing discussion, the proximal and distal tightening assemblies 22, 23 are provided to incrementally tension the first and second force straps 18, 20, and selectively allow release of tension in the force straps 18, 20.

A proximal stability strap 28 is secured to the medial side of the brace 10 and extends to the lateral side whereat it is connected to a proximal buckle assembly 32 that is connected to the sleeve 12. A distal stability strap 30 is secured to the medial side of the sleeve 12 and extends to the lateral side whereat it is connected to a distal buckle assembly 33 which is also connected to the sleeve 12.

According to this embodiment, each of the stability straps 28, 30 includes a cushion feature 34, such as foam or a textile pad that is secured thereon for enhanced rotational prevention and additional comfort. The stability straps 28, 30 each have an adjustment feature 35, such as a hook and loop fastener system, to enable adjustment of the length of such straps 28, 30. Moreover, the cushion feature may include a frictional feature (not shown), such as a pattern of deposited silicone, rubber, or a mildly abrasive material. In addition, the cushion feature may be breathable, and have a construction similar to the spacer elements described below.

In one variation, the stability straps may be releasably secured to the knee brace. For example, the stability straps may include a snap fastener element that corresponds to a snap fastener element supported by shells of the knee brace. In another variation, other suitable releasable fasteners may be used to permit installation and removal of the stability straps from the knee brace.

An embodiment of the knee brace may be provided alternatively with one force strap connected to a tightening device and another strap that is adjustable with a fastener system such as hook and loop fasteners. For example, in the event that it is desired to provide a low profile brace, one could use a force strap system that is connected to and adjustable at the proximal portion (corresponding to the femur of the wearer) of the brace that includes a tightening device, whereas the force strap system connected to the distal portion (corresponding to the tibia of the wearer) may simply use a hook and loop fastener system. Variations of this embodiment are also useful in order to mitigate issues of a tightening device extending over pressure points that may be present over the tibia.

The embodiment of the brace of Figs. 3-6 is generally oriented to relieve lateral compartmental osteoarthritis of a left knee. This brace may be configured to treat medial compartmental osteoarthritis of the left leg or, in the alternative, medial or lateral compartmental osteoarthritis of a right knee. The reconfiguration for treating medial compartmental osteoarthritis comprises arranging the force straps in a reverse configuration so the force straps have an intersection point on the proximal-distal plane on the posterior-medial side of the device.

Turning to Fig. 3, the internal features of the embodiment of the brace 10 are shown in greater detail without the sleeve 12. Of interest are the proximal and distal shells 40, 42 which provide the structure for brace, and connect to the force straps 18, 20 and the stability straps 28, 30. Of additional interest are the proximal and distal spacer elements 46, 48 which provide anti-rotational means, such as a frictional feature, and cushioning for the brace 10 when worn on a leg.

The proximal and distal shells 40, 42 are configured for placement between the lateral and medial sides of an anterior portion of the brace 10. Similarly, the proximal and distal spacer elements 46, 48 are configured with a shape generally corresponding to the proximal and distal shells 40, 42, and are arranged for connection to a rear portion of the sleeve 12 in register with the shells 40, 42. It is desirable that the proximal and distal shells 40, 42 be in register with the proximal and distal spacer elements 46, 48 so that as the force straps and stability straps are tensioned about a leg. The spacer elements 46, 48 are urged against a leg so as to prevent rotation of the brace 10 due to the forces applied to the leg from the force straps.

According to variations of the shells, they may be configured for placement on the posterior side of the brace, or at least have sections that extend over a portion of the posterior section of the brace. In addition, variations of the shells may involve one shell such as the proximal shell extending about the anterior side of the brace between the lateral and medial sections, whereas the distal shell extends over the posterior side of the brace and further includes a segment wrapping over at least one of the lateral and medial sides to cover a portion of the distal-anterior section of the brace.

A benefit of the spacer elements in a hinge-less knee brace is that these spacer elements prevent migration of the shells towards one another. The spacer elements also maintain the knee brace on the user's leg due to anti-rotation means. Moreover, the spacer elements can also resist any rotational forces that may be applied by the force straps.

### ii. Method of Applying the Knee Brace

In operation, the embodiment of the brace according to Figs. 3-7 is attached to the user, for whom it may be custom made or pre-fabricated, by positioning the device on the leg with the center portion of the sleeve placed over the anterior knee. The proximal and distal force straps 18, 20 are positioned above and below a side of the knee, and tightened accordingly. This arrangement of the force straps ensures that the force straps tighten above and below the knee as the leg moves into extension and loosens as the leg moves into flexion. The tightening of the force straps 18, 20 during extension of the knee prevents movement of the bone upon extension of the leg, and thus treats the adverse affect of compartmental osteoarthritis.

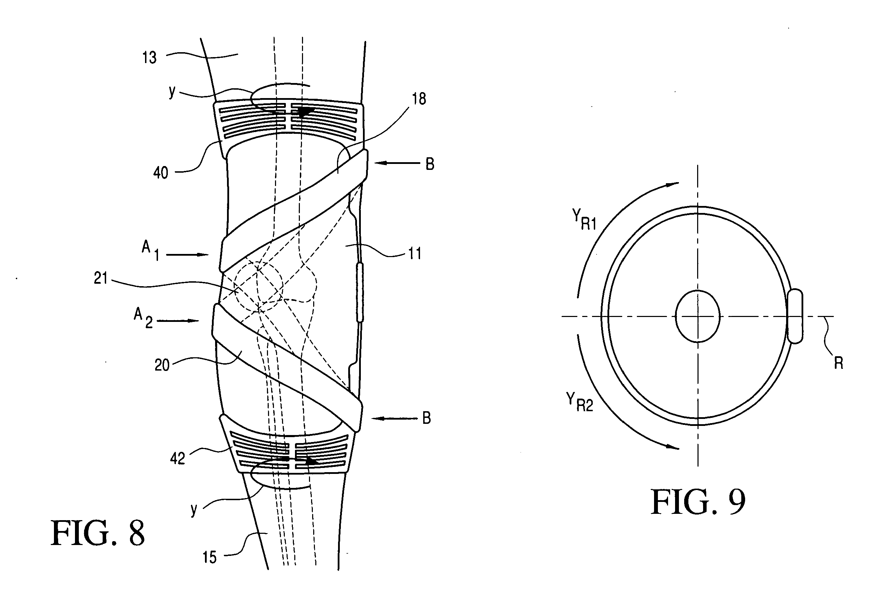

Figs. 8 and 9 illustrate the brace on a left leg 11 that defines proximal and distal portions corresponding to the femur and tibia, respectively. The tightening of the force straps 18, 20 tend to depressurize the compartment of the knee by increasing the space between the bones on the affected side of the knee. The configuration of the force straps along the frame elements 40, 42 provides reaction points for the force straps 18, 20. Thus, tightening of the force straps 18, 20 causes the frame elements 40, 42 in combination with the spacer elements to stabilize the knee on the side opposite the intersection area 21.

From Fig. 8, the forces A₁ and A₂ are shown applied to the medial side of the knee at a greater degree than the single force A generated by the prior art braces, as exemplified in Figs. 1 and 2, due to the greater distribution of pressure on the leg. Additional forces B are applied on the lateral side of the leg approximately where the force straps are attached to the proximal and distal members. By applying two forces, these forces counteract to mitigate the rotational moment that is present in the prior art braces wherein rotational forces Y_{R1} and Y_{R2} are generally equal to and cancel one another.

It has been found that if only one force strap is used without any intersecting points, as in the prior art braces, the skin and soft muscle tissue move with the shells. As a result, the unloading effect of the straps decreases significantly. By using the two force straps to form forces A₁ and A₂, rotation of the device on the leg is reduced and effectively prevented. This provides a sufficient unloading effect by the brace on the knee.

The force straps are substantially inelastic since, as mentioned previously, it has been found that in prior art braces that employ elastic force straps, the knee and leg counteract the suppleness of the elastic straps thereby reducing the unloading effect on the knee. By using substantially inelastic force straps, the knee is unable to resist the straps and, consequently, a greater unloading effect is obtained of the knee.

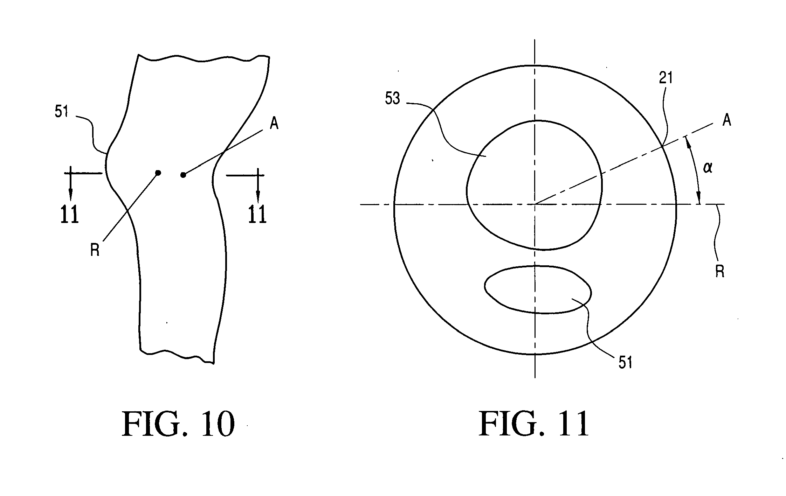

Referring to Figs. 10 and 11, the resultant force "A" of forces A₁ and A₂ in Fig. 8 is applied as the knee goes into extension. The force straps preferably cross at intersection point 21 at angle α ranging about 5° to 20° posterior of the normal axis of rotation of the knee with the knee cap 51 being in front of rotational axis R and tibia 53. The intersection point is preferably not the point of unloading; instead, the unloading point is directly on the lateral side for the medial brace, and directly on the medial side for the lateral brace.

The knee brace may be tailored to optimize the forces generated by the force straps. When the brace includes two force straps or has a single force strap with two intersecting portions, a greater moment is applied to a leg providing that the same force is now applied by two force straps. This results in a lower angle that may be used to configure the force strap(s), and consequently proximal and distal frame members or shells may be positioned closer together as opposed to in prior art braces having only a single force strap which spirally extends once between proximal and distal members.

### iii. Spacer Elements

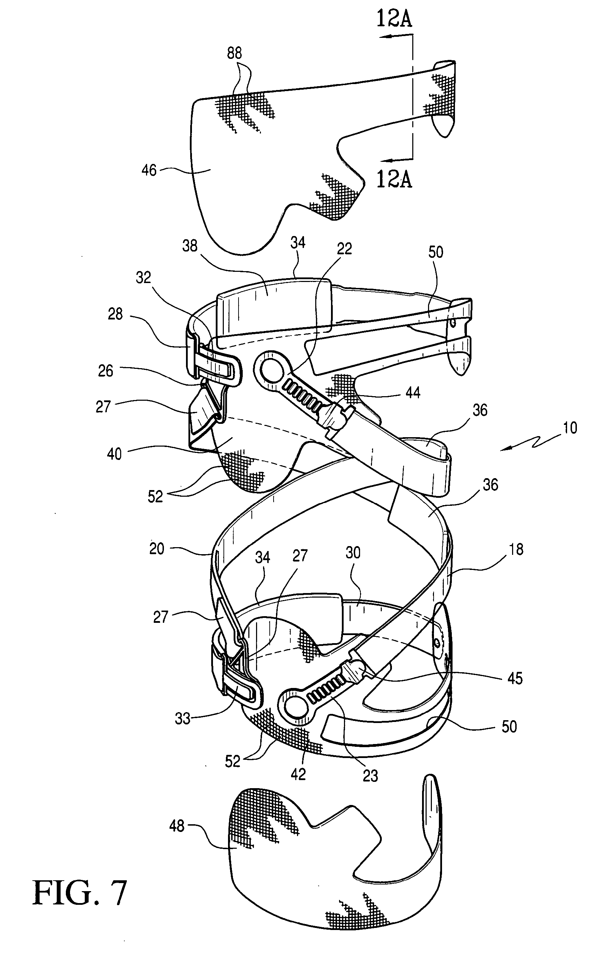

As shown in Fig. 7, the brace 10 includes proximal and distal spacer elements 46, 48 that are contoured in a similar configuration as the shells 40, 42. These spacer elements 46, 48 are arranged so as to be breathable by permitting a free flow of air therethrough. The spacer elements also preferably include a friction feature, as in a frictional layer, on at least one side thereof.

The spacer elements may be connected to the sleeve via removable means, such as with a hook and loop fastener system, or may alternatively be secured to the sleeve via stitching, adhesives, or other similar fastener features. While the spacer elements are intended not to interfere with the motion of the knee, they are intended to provide sufficient frictional force and cushioning to maintain the shells against the knee due to the vertical forces created by the force straps.

According to this embodiment, the spacer elements 46, 48 are secured to the posterior side of the sleeve 12. The spacer elements 46, 48 have a breathability feature 88. According to a variation, the breathability feature comprises a pattern of openings defined across the spacer elements 46, 48. Alternatively, the breathability feature may comprise a breathable fabric, and may be combined with a pattern of perforations to further enhance the breathability of the spacer elements. Moreover, the spacer elements may be constructed from a material that provides cushioning and further compresses, at least in part, when the brace is worn.

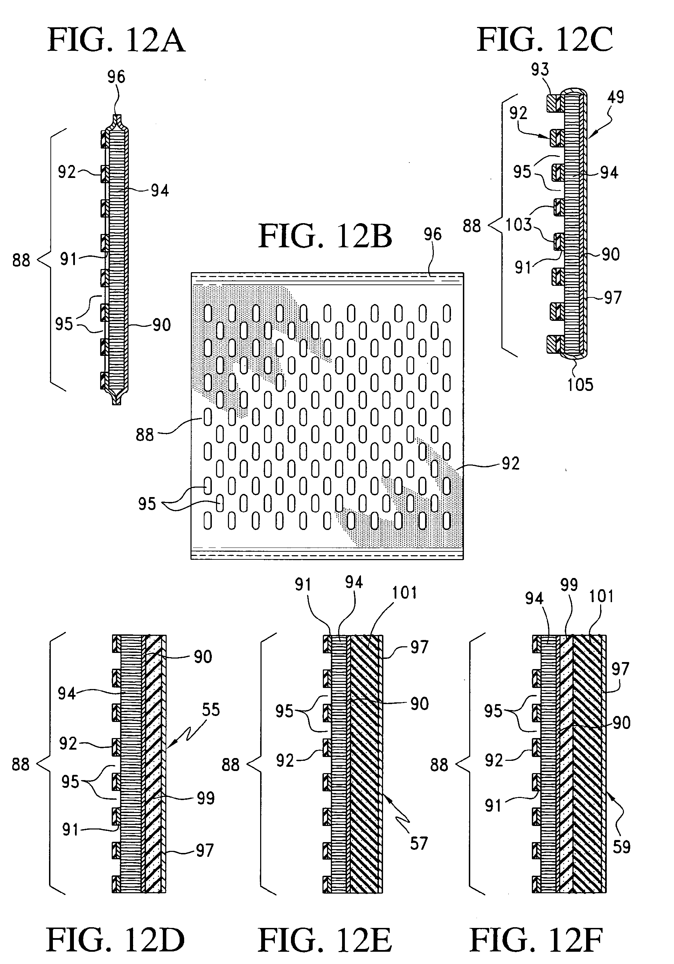

According to one variation of the spacer element 47 exemplified in Fig. 12A, which is a cross-section of the spacer element 46 in Fig. 7, the spacer element 47 preferably has a breathability feature defined by an apertured friction feature in the form of a frictional layer 92 that is provided on an apertured first surface 91 of a ventilated and compressible core 94. The frictional layer 92 preferably has a high frictional coefficient against the skin or clothing.

Through the apertured first surface 91 and an opposed second surface 90, the core 94 permits the passage of air therethrough, and yet is compressible to provide adequate cushioning and securing to a leg or other anatomy. In addition, the spacer element has a reinforced edging 96 protecting the core portion, and enhancing the durability of the spacer element. The reinforced edging may comprise a material separate from the core or frictional layer, such as a hook-receivable material, as explained in detail in connection with the variation of Fig. 12C.

In observing Fig. 12B, the breathability feature 88, located on at least on one side of the spacer element 47, is defined by the pattern of apertures 95. Preferably, the apertures 95 of the frictional layer 92 and the apertured first surface 91 coincide with one another so that they are common with one another as exemplified by the apertures 95 in Fig. 12B.

In this variation, the frictional layer 92 is generally discrete resulting in a distinct and separate layer. The frictional layer may be free standing so as to be applicable onto the first surface of the core as an already-cured sheet, or may be deposited or formed onto the first surface of the core so as to be laminated or alternatively coated onto the first surface of the core. The frictional layer may be secured to the core through bonding with an adhesive, lamination under suitable heat and pressure, or coating of the core with uncured or partially cured compositions.

An exemplary method may be adapted from U.S. patent 7,161,056. According to this method, an uncured silicone composition is molded to a particular configuration in the form of a layer at a suitable curing temperature. After a period of curing, the thus formed partially cured silicone composition layer is applied to or pressed against a substrate, and is continuously cured until curing is complete. By molding the frictional layer at least partially prior to application onto a substrate, one can tailor the shape of any apertures or pattern that the frictional layer may take.

According to this variation, the frictional layer 92 is generally continuous so that it forms a web-like structure. The apertures 95 of the frictional layer 92 may be formed in a predetermined pattern that may or may not be independent from the apertures or any ventilation feature of the core. In alternative variations, the frictional layer may be provided in a pattern of distinct segments and locations, so that it is not continuous as in the web-like structure. Such segments may comprise lines, dots or other individual shapes.

It will be noted, however, that the apertures of the frictional layer and the core, as either with or without the first surface, must not necessarily coincide with one another. For example, the core may be open-cell foam having a plurality of random pores located along the surface thereof. The frictional layer, on the other hand, may have a pattern of uniformly spaced apertures independently provided from the pores of the foam. What is important is that at least some apertures of the frictional layer and the core intersect so as to permit the transfer of air through the spacer element.

An example of providing a frictional layer over a core such that the apertures of the frictional layer do not correspond to apertures or pores of the core is found in U.S. patent 7,161,056. It will further be pointed out that the frictional layer of this invention may be applied to non-apertured substrates for particular applications wherein it is not necessary that the substrate be breathable but wherein it is desirable that there is some form of frictional resistance provided on the substrate.

In another variation of a spacer element 49, as shown in Fig. 12C, the second surface 90 of the core 94 is secured to a hook-receivable material 97. This enables the spacer element to be easily secured to corresponding hook material with may be secured to the shell of a brace. Also, by providing hook-receivable material on the spacer element 49, the surface of the spacer element is soft to the touch, and enables the spacer element 49 to be used alone as a bracing support. The hook-receivable material may be any type of hook-receivable material known to one skilled in the art.

The spacer element 49 may allow for the hook-receivable material to effectively form the seam 96 in Fig. 12B, so as to cover the edges of the core. Examples of hook-receivable are well known to those skilled in the art, and include materials such as broken and unbroken loop material.

Fig. 12C also exemplifies how the frictional layer 92 may include first areas 93 that have greater thicknesses than other (i.e., second, third, etc.) areas. These first areas 93 may have variable thicknesses when it is desired that there is greater cushioning or frictional support, as demonstrated by Fig. 12C by the gradually thicker first areas 93 towards the middle portion of the substrate. Further, such variable thicknesses may be advantageous in embodiments that do not include an apertured substrate, or a substrate that does not include nor has only minimal cushioning properties. As such, the thicker first areas 93 may be adapted so as to provide both the required frictional resistance and cushioning or suspension of the substrate against an object (as in the anatomy of the wearer of the substrate).

It will be noted that the frictional layer may include at least two layers of its own. For example, a top area (or layer) 103 may be a softer or compressible silicone composition, whereas the base first area (or layer) 93 may be more rigid and less compressible. This allows for the adaptation of the frictional feature to be tailored to certain anatomy that may be more distressed or sensitive, and further vary in softness across its pattern which may correspond to certain regions of the substrate upon which it is applied. An example of using and forming different layers of silicone compositions is found in U.S. patent 6,136,039.

Fig. 12C also exemplifies another feature of the frictional layer in that the cross-section of the layer may vary as shown by the rounded edges 105. This configuration provides a transition of the frictional layer across the skin, thereby easing the application to and removal from skin. The rounded edges 105 may also allow the frictional layer to compress and deform without occluding any or some of the openings or ventilation of the core or other substrate. The rounded edges 105 may also provide aesthetic attributes allowing for both ornamental and functional patterns of the frictional layer. The rounded edges 105 may be formed during the normal course of forming the frictional layer.

In another spacer element variation shown in Fig. 12D, the spacer element 55 includes a foam layer 99 secured to the second surface 90 of the core 94. This foam layer 99 preferably has greater rigidity than the core 94, and is ventilated and compressible as in the core 94. A hook-receivable layer 97 may be secured to a surface of the foam layer 99.

In another spacer element variation shown in Fig. 12E, the spacer element 57 includes a thicker foam layer 101 than the foam layer 99 used in the spacer element 55. The thicker foam layer 101, while adding to the overall thickness of the spacer element, prevents shifting or wiggling of the spacer element 55. In other words, it makes the spacer element 57 more rigid. Again, as in the foam layer 99, the foam layer 101 is ventilated and compressible.

In another spacer element variation shown in Fig. 12F, the spacer element 59 includes both foam layers 99 and 101. The multiple foam layers enables taking advantage of different properties of the foam, such as different rigidities, porosities, and densities. The hook-receivable material and the foam layers 99 and 101 may be secured to the core and to each other by using a heat activated adhesive in a lamination process.

According to Figs. 12G-12L, another embodiment of a spacer element 700 is shown. The spacer element 700 is contoured to correspond to a shell or frame structure or assembly of an orthopedic or prosthetic device, or may be provided with straps 703 and serve as an orthopedic device itself. The spacer element 700 defines regions having different thicknesses 702, 704, 706, 708. Indeed, while this spacer element may be formed as a laminated structure and define a cross-section much like the other embodiments and variations of a spacer element described herein, this particular embodiment is arranged to have varying degrees of compressibility and flexibility.

As can be observed from Fig. 12G, various channels, indentations and grooves can be molded into selected regions of the spacer element for accommodating brace hardware, anatomical contours, or to alter the stretch characteristics of certain regions of the spacer element. At least some of the layers of spacer element are compression molded via heat and pressure in specific areas to alter the thickness and/or density of the material in those areas in order to improve the function of the brace.

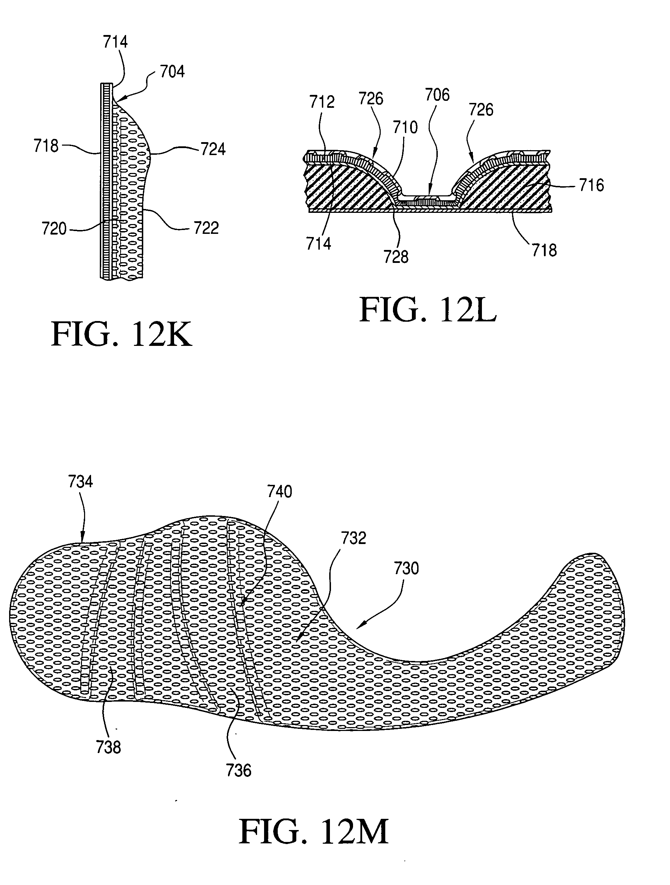

A method for manufacturing the spacer element having contours and compression molded thickness variations of the type depicted in Fig. 12G and Fig. 12M may be derived from U.S. patent 5,695,452.

Turning to specific features of the spacer element according to this embodiment, Fig. 12H shows a cross-sectional view of the elongate center portion of the spacer element. The center portion 702 includes a peripheral edge region 704, a transitional thickness region 708, and a central thickness region 702. The regions demarcated by the different thicknesses are achieved by varying the thickness of the foam layer 716 or are due to an absence of the foam layer 716 at particular regions.

The spacer element 700 includes a frictional layer 710 and a core 712 of the type and structure described above in connection with other embodiments and variations. The spacer element 700 also includes a first layer 714, which may be textile-based, located between the core 712 and the foam layer 716, as well as a second layer 718 which may be hook-receivable. The second layer is preferably continuously flat and devoid of any indentations or protrusions and forms an entirety of a first outer surface of the spacer element.

At the center region 702, the spacer element includes all of the aforementioned layers, wherein the foam layer is either slightly compressed or not compressed at all. This arrangement allows for a maximally compressible region at the center region whereat most of the padding support is required for the wearer of the spacer element.

As for the transitional region 708, the foam layer 716 is locally compressed with the compression increasing toward the peripheral edges of the foam layer 716. The transitional region, while compression molded, generally retains its molded thickness (accounting for some compression when worn) and shape while and after the spacer element is worn against the anatomy of the wearer. This region is anatomically configured to minimize compression of the spacer element, to provide greater rigidity to the spacer element at least along its edges, and to ease pressure on the wearer.

The peripheral edge region 704 lacks the foam layer and has areas of compression provided by the core 716.

It will be noted that the spacer element of Fig. 12G has first and second surfaces which correspond to opposed sides of the spacer element. The first surface is substantially flat and is covered in its entirety by the second layer 718. The second layer is preferably hook-receivable so as to engage a bracing component and be directed away from the wearer.

The second surface is preferably arranged to be worn against the wearer. The contours of the spacer element protrude outwardly relative to the first surface, and are formed along the second surface of the spacer element. The entirety of the second surface is covered by the frictional layer 710 and defines a plurality of apertures 720, as depicted and also discussed in connection with the other embodiments and variations described herein.

Fig. 12I depicts the transitional region 710, the peripheral edge portion 704 and a border 719 between the two regions. At the border 719, the core 712 has a reduced thickness in which the core 712 is pinched relative to portions of the core outside of the border 719.

Fig. 12J depicts a flexible region 706 of the spacer element whereat the spacer element does not include the foam layer 716, as shown in Fig. 12H. The layers in Fig. 12J include all but the foam layer 716 in Fig. 12H, and may be compressed, in particular the core 712.

It will be noted that the entirety of the spacer element may be flexible, with the flexible region having increased flexibility over other regions of the spacer element.

In reference to Fig. 12K, a section of the spacer element is shown as having additional areas of varying thickness. For example, a concave region 722 is provided which may correspond to a particular anatomical region so as to avoid a raised portion and to better embrace the anatomy. Bordering the concave region 722 is a convex region 724 which may surround at least in part the concave region 722. Between the concave and convex regions 722, 724, the thickness of the foam layer (not depicted) may vary. While the convex region 724 is shown as constituting a peak, it may constitute a flattened or planar region, much like the central region 702, as shown in Fig. 12H.

Fig. 12L shows how the flexible region 706 is located between opposed concave or fillet regions 726 of the spacer element which transition to a central region of the compressible material layer having a predetermined thickness. The compressible material layer 716 in the fillet regions 726 has a variable thickness and terminates at peripheral edges 728 of the flexible region 706.

Turning to Fig. 12M, the depicted spacer element 730 is formed in a manner that corresponds to the shape of a shell 448 that may be used with the knee brace of Fig. 3, and a represents how the spacer element may be contoured in accordance with frame elements in an orthopedic device. The spacer element 730 is formed similarly to the embodiment of Fig. 12G in that it has flexible regions 736, 738 which may be formed as the flexible region 706, yet each of these flexible regions 736, 738 may have varying degrees of flexibility relative to one another by way of the core being compressed. The spacer element 730 also includes a central region 732, a peripheral edge region 734 and transitional region 740. Additionally, the thickness of the central region 732 may be formed in a similar manner to the thickness changes described in connection with Fig. 12K.

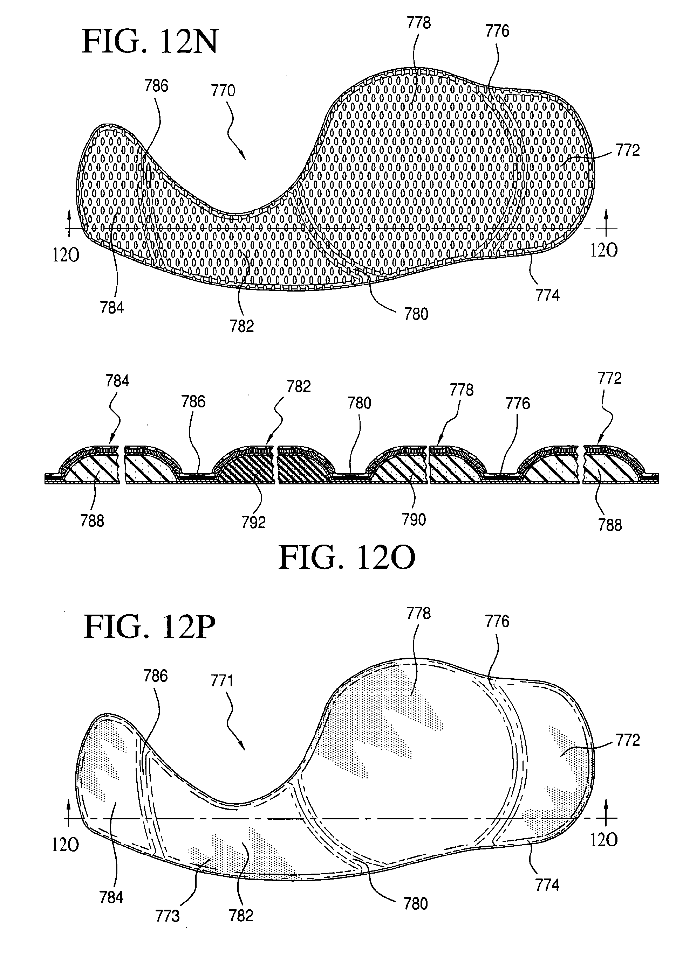

Fig. 12N illustrates yet another variation of the spacer element 770 having an exemplary shape similar to the shape of the spacer element 730 in Fig. 12M. The spacer element 770 bears a similar cross-sectional structure to the structure of the spacer element in Fig. 12H with the exception of the firmness of the foam.

In this variation, the spacer element 770 has padding layers of different foam firmness according to particular anatomical regions upon which the spacer element is intended to be placed against the wearer. For example, the spacer element 770 includes opposed end portions 772, 784 which have a lower firmness than first and second middle portions 778, 782. Particularly, the middle portions may likewise have different firmness relative to one another, such that the second middle portion 778 has a greater firmness than the first middle portion 782. Each of the portions may be separated by a flexible region 776, 780, 786.

As depicted in Fig. 12O, the padding layer 788 for the opposed end portions 772, 784 has a lower firmness than the padding layers 790, 792 used in the first and second middle portions 778, 782, respectively. The flexible portions 776, 780, 786 are substantially devoid of the padding layers so as to increase their flexibility. The padding layer firmness of the portions may be achieved by using foams having a cellular structure that allows the foam to compress and recover in response to loading or applying the spacer element onto anatomy of the wearer, and unloading or removing the spacer element from the wearer. In addition to the inherent properties of selective foam, the portions can likewise be mechanically thermoformed in accordance with the other variations of a spacer element described herein.

Fig. 12P depicts another embodiment of a spacer element 771 having a smooth layer (such as a doeskin, microfiber or suede layer known to the skilled person) which is intended to be adjacent the skin of a wearer. The spacer element 771 is formed substantially the same as the spacer element 770 depicted in Fig. 12N with the exception of the replacement of the apertured layer upon which a frictional layer is located with the smooth layer. It is preferable that the smooth layer is pliable and breathable, and is laminated upon the adjacent layer on which it is located. The smooth layer is particularly defined as having minimal frictional properties when rubbed against the skin of a human being, and preferably slides over the skin of the wearer with ease.

In connection with any of the orthopedic brace or knee brace embodiments described herein, the smooth spacer element may be used to replace any of the spacer elements described herein. According to one example, the smooth spacer element, as in Fig. 12P, may take the form of an upper pad connected to an upper frame element and has an exterior surface adjacent to the upper frame element and an interior surface opposed from the exterior surface. The interior surface of the upper pad is smooth to human touch so as to provide low resistance to movement when pressed against skin of a wearer of the brace.

The knee brace may also include a frictional spacer or a lower pad connected to the lower frame element and having an exterior surface adjacent to a lower frame element and an interior surface opposed from the exterior surface. The interior surface of the lower pad has a frictional coating of the types described herein and provides substantially more resistance to movement of the leg of the wearer than the interior surface of the upper pad when pressed against skin of a wearer of the brace.

This particular embodiment of the knee brace with the smooth upper pad is provided to allow an upper frame element to piston on the thigh of the wearer without irritating the skin of the wearer. The lower frame element, however, is held firmly against the shin or tibia portion of the wearer due to the substantially frictional surface of the lower pad. The upper pad therefore allows the upper frame element to accommodate movement of the thigh, whereas the lower pad holds the lower frame element (in combination with at least one strap of any type described herein) firmly against the shin of the leg such that the lower frame element does not shift upon movement of the leg.

In another variation, the spacer elements may be directly connected to the shells. Of course, such spacer elements may be configured so that they may be removed from the shells and reapplied without harming their structure. Fasteners such as hook and loop systems may be used to attach the spacer elements to the shells.

Preferred substrate materials that may be used to form the spacer elements include open or closed foams, neoprene, and textiles. For example, some of the materials could be disposable non-woven materials, neoprene or neoprene replacements (i.e., airprene or COOLMAX), perforated closed cell foams (ethylenes, eva cross-links or plastizote), circular knits, and stockings.

While numerous materials may be used as the spacer elements, according to certain embodiments it is desirable that these materials have a three-dimensional knit structure covered by a mesh that provides sufficient breathability, insulation, compression, durability, and recovery. An exemplary material is produced by Gehring Textiles under product numbers SHR 701, SHR 714 or SHR 754F.

According to this embodiment, the posterior surfaces of the spacer elements are coated with the frictional layer which comprises a continuous, discrete layer of cured silicone elastomer material 92. The silicone elastomer material is preferably sufficiently thick and soft to be comfortable to the user, and yet provides a seal between the proximal and distal members, and the skin or clothing of a user of the brace 10.

As exemplified in Fig. 12B, the silicone layer preferably has apertures which correspond to apertures of the mesh surface of the three-dimensional fabric when such a fabric is used to form the substrate of the spacer elements. In a variation, the friction feature may comprise a plurality of silicone-based dots, lines or other patterned arrangements which are deposited to the surface of the substrate of the spacer elements.

An advantage of using silicone as the frictional layer is that it may be molded to accommodate a variety of different substrates, either apertured or non-apertured. The pattern of the silicone frictional layer may be molded to correspond to irregular apertured configurations, whether they are slots, circles, squares, and other shapes, and possess varying alternatives. Moreover, the silicone frictional layer may be bonded, or directly laminated or coated onto substrates thereby providing substantial manufacturing flexibility for various substrates upon which the frictional layer is provided.

The silicone material may be mixed with scenting, anti-inflammatory, anti-bacterial, and coloring agents. Moreover, the silicone material may include skin friendly agents such as aloe vera or Vaseline. A more complete description of additives to the silicone may be found in U.S. Patent 6,485,776.

The silicone material preferably has a Shore hardness of 25-70; a minimum tensile strength of 230 lbs/inch; a 100% modulus of 8 psi; a 500% modulus of 61 psi; minimum tear strength of 49 lbs/inch; a maximum strength of 500 lbs/inch and an elongation of about 1000%. The silicone material may be disposed in a uniform thickness in both circumferential and longitudinal directions, or may have a varying thickness to accommodate varying shapes of a body portion, protrusions, contours, etc.

It will be understood that a silicone-based frictional layer is provided as merely an example of a frictional layer. Other types of frictional layers may be provided such as those based on thermoplastic elastomers or rubbers such as styrenic block copolymers, thermoplastic polyolefins, thermoplastic vulcanisates, themoplastic polyurethanes, thermoplastic copolyesters, melt processable rubbers, and thermoplastic polyether block amides. Further, it is considered within the scope of the invention to use expanded polyester foams formed of a web-like pattern as the frictional feature.

Many of the aforementioned materials may be shaped into appropriate webs or layers that may be bonded, laminated or coated onto a substrate in order to provide a frictional feature. Moreover, as described in connection with the embodiment shown in Fig. 12C, these frictional features formed from these materials may be formed so as to have areas of varying thickness or of a thickness that are sufficient to eliminate or minimize the need for a padded substrate.

While the spacer elements are preferably shown with a frictional feature provided on their posterior surfaces, the anterior surfaces of the spacer elements may likewise be coated so as to frictionally couple with the sleeve. In the alternative, the spacer elements may be constructed of sections of elasticized fabric and coated with silicone of the type described in U.S. Patent 6,592,539.

In a variation of the brace, the brace does not include the spacer elements and instead relies on the proximal and distal member straps for attachment to a user. In another variation, the spacer elements are not coated with silicone. These spacer elements provide a compressive buffer between the proximal and distal members and a leg of a user of the brace. In yet another variation, the substrate may comprise a textile having superior frictional properties, and as a result, merely the compression of the textile is sufficient to prevent rotation of the brace when worn on a leg.

In yet another variation, the spacer elements may comprise a silicone mesh comprising a textile that is impregnated with silicone. This silicone mesh defines a pattern of apertures that permit the transport of air therethrough. In yet another variation, the spacer elements form a silicone sheet having a pattern of apertures, and sufficient thickness to serve as a buffer between the sleeve, frame members, and a leg.

A feature particular to these spacer element embodiments is that the apertures in the silicone layer and at least the first layer of the three-dimensional fabric are preferably vertically oriented, as depicted in Fig. 12B. Specifically, a length of the apertures is vertically oriented, for example by way of the generally elongate apertures 95 depicted in Fig. 12B.

The vertical orientation of the apertures allows the spacer element to bend without wrinkling or bunching, thereby the spacer element has a generally smooth inner surface (surface adjacent the anatomy of the wearer) when the spacer element is shaped or bent about anatomy of the wearer. It is noted however that the apertures may be oriented in a variety of configurations and are not limited to only being oriented in a vertical configuration, as depicted in Fig. 12G wherein the apertures are generally horizontally oriented.

As with any of the spacer element embodiments and variations described herein, these spacer elements may be used in a variety of orthopedic and prosthetic applications where frictional control, breathability, compression or padding is required or desirable. The spacer elements may be used in combination with any sort of bracing or frame members, or may be used alone with or without strap or securing means.

The embodiments and variations thereof of the spacer element described herein may be used in a variety of applications in addition to those related to orthopedic and prosthetic uses. For example, the spacer elements may be adapted for use in protective gear wherein there is a need for devices that enhance suspension, rotation-resistance and breathability. Also, the spacer element may be used in intimate apparel to provide breathability, comfort and suspension. A layer of the frictional feature may be applied to gloves for additional grip. Foot apparel so as to provide ventilated and frictional means to prevent shoe slippage and to further enhance comfort. Other applications not mentioned above and requiring comfort, breathability and resistance to rotation or movement are considered to be embraced by the spacer element of this disclosure.

### iv. Additional Features

Additional features may be used in connection with the aforementioned embodiments of the knee brace.

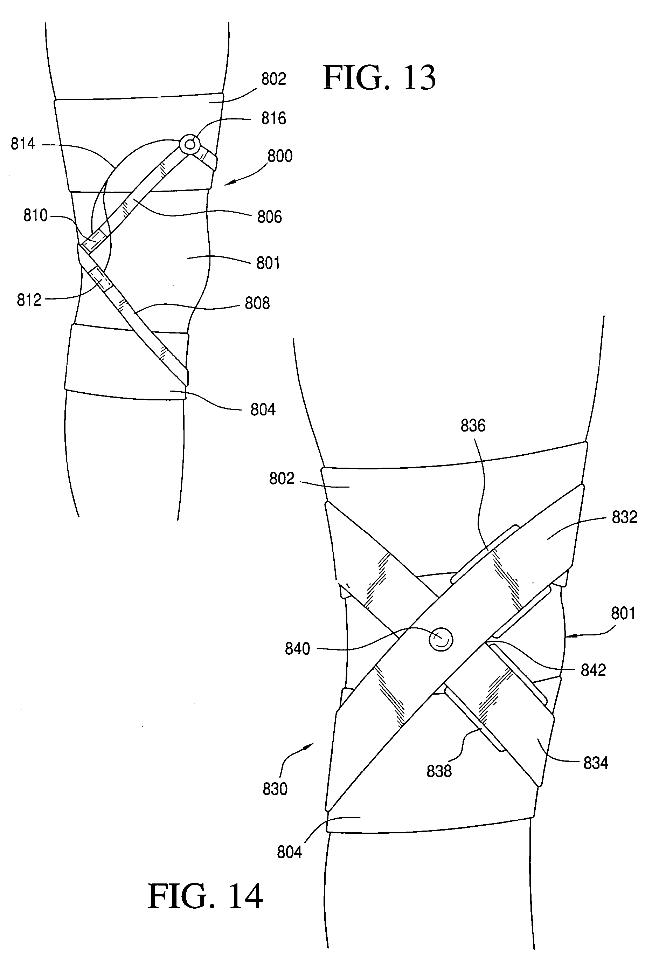

One such feature includes load cells that are connected to force straps to measure the force exerted on a knee. According to this feature, as exemplified in Fig. 13, an orthopedic device 800 exemplified as a knee brace includes frame elements 802, 804 load cells 810, 812 are connected to the first and second force straps or strapping system elements 806, 808. These load cells 810, 812 monitor the pressure applied on a knee 801 and relay via connection(s) 814 a pressure reading to a tightening device 816 of any type described herein. The tightening device 816 according to this embodiment may be equipped with a drive motor (not shown) that incrementally adjusts the tightening device by either tightening or releasing the force straps in accordance of any of the types of strapping systems, cables, and ratcheting systems described herein.

Of course, this embodiment is not limited to requiring two load cells, and one or multiple load cells may be used to determine the pressure on the knee caused by the force strap.

This feature of the tightening device is particularly advantageous since it permits precise tension adjustment of the strapping system to treat a specific user. The predetermined parameters include a range of dosage requirements for users. These dosage requirements include forces required for a user to unload the compartmental osteoarthritis of the knee. For example, one dosage would equal about 3Nm of unloading. The maximum unloading, in this example, is 12Nm so 4 doses would provide a maximum unloading of the knee. The load cells may be configured for a user during a fitting process by an orthotist who could establish a dosage requirement for the user.

In another variation, the load cells may be integrated with the knee brace and the tightening device. The data obtained by the load cells can then be used by the tightening device to change the tension in the force strap during a gait cycle. According to this variation, an accelerometer device is required to determine the stage of the gait at a particular point in time. This can be particularly useful when walking up or down ramps or hills, or going up or down stairs since the knee is bears weight when in flexion so that the strap is pulled tighter during such stages of walking.

In another feature that may be used in combination with an orthopedic device exemplified as a knee brace 830, a shown in Fig. 14, an inflatable bladder system for providing additional cushioning and fitting of the force straps and stability straps, or strapping system. As illustrated, the force straps 832, 834 depend from frame elements 802, 804 and are provided with a plurality of bladders 836, 838 connected to a pump 840 to provide relief to a leg. The bladders 836, 838 are particularly positioned on the force straps 832, 834 at locations proximal and distal of the leg whereat the force straps apply the maximum pressure on the knee 801, and are at least near a junction 842 formed by the straps 832, 834.

In operation, the force straps are applied over the knee with slight tension. As the bladders are inflated, the force straps tighten over the knee due to the increase in size of the bladders. The pump permits inflation and deflation of the bladders. The pump may be integrated with the force straps or be located remote therefrom.

Examples of pump and bladder systems that may be used in combination with the force strap of the knee brace are described in U.S. Patents 5,022,109 and 6,598,250.

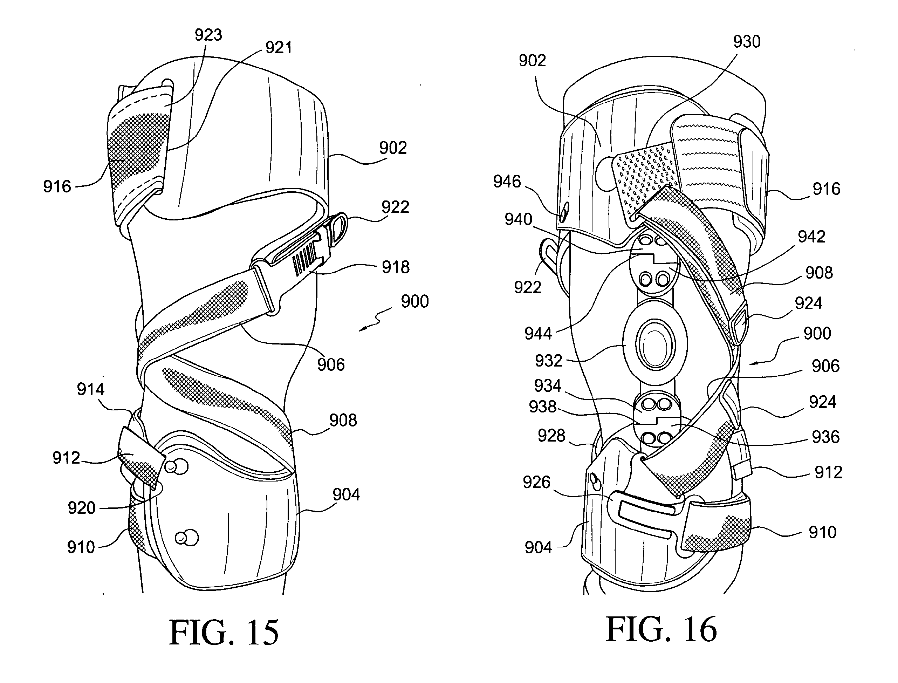

Figs. 15 and 16 exemplify another embodiment of an orthopedic device or knee brace having various strap adjustment features, strap pads, and feature location matching indicia.

Figs. 15 and 16 illustrate the orthopedic device 900 as having upper and lower or proximal and distal frame elements, members or shells 902, 904. As with other embodiments described herein, the device includes first and second force straps 906, 908 connecting to the shells 902, 904. A tightening device 918 having a flexible pull tab 922 is used to adjust the second force strap 908. relative to the upper frame element 902.

A stability strap 910 is provided at a lower portion of the lower frame element 904 such that it extends to first and second sides of the lower frame element 904. A gastroc strap 912 is provided above the stability strap 910, such that the gastroc strap 912 carries a gastroc pad, and extends to the first and second sides of the lower frame element 904. The gastroc strap 912 is adjustably secured to the second side of the lower frame element via a slot 920 formed by the lower frame element 904.

As depicted, the lower frame element 904 extends over the anterior portion of the lower leg LL, whereas the stability strap 910 and the gastroc strap 912 extend generally laterally relative to the length of the leg, and are placed over the posterior portion of the lower leg.

A thigh strap 916 secures to opposed sides of the upper frame element 902, whereby the thigh straps extends generally laterally relative to the length of the leg, and is placed over the upper leg UL, in particular the posterior side of the thigh. The thigh strap 916 is adjustably secured to the second side of the upper frame element 902 via slots (at least one) 925, 927, through which pull sections 921, 923, respectively, of the thigh strap 916 extend.

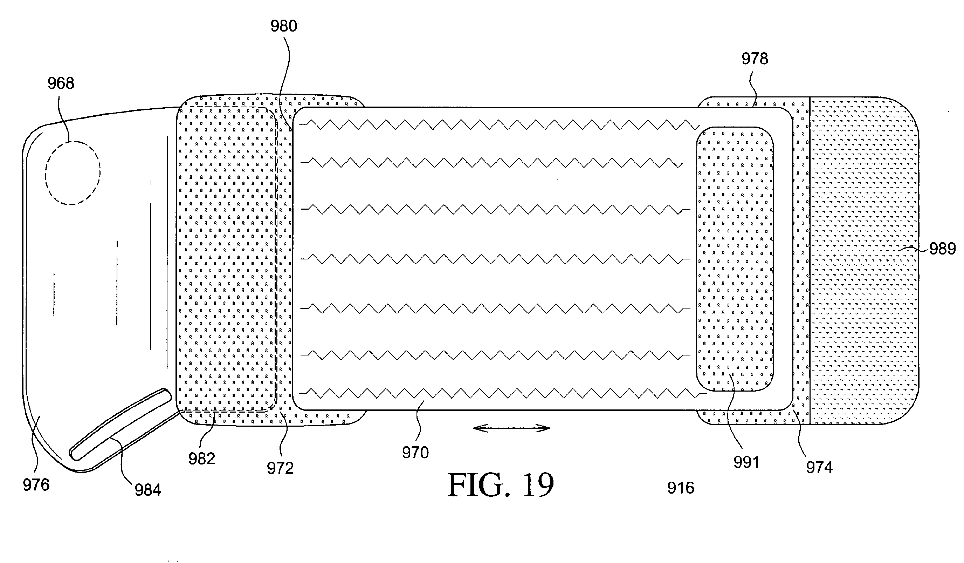

The knee brace 900 includes a lower buckle assembly 926 having a buckle 928 similar to other embodiments described herein. The knee brace also includes an upper bracket assembly 930 discussed in more detail in connection with Fig. 19. The upper bracket assembly 930 secures the thigh strap 916 to one side of the upper frame element 902.

The knee brace 900 includes a hinge 932 similar to the hinge provided in the Unloader Spirit brace sold by Ossur hf. This depicted hinge 932 accommodates a wide range of varus/valgus adjustments. Variations of the hinge are found in U.S. patent application 12/264,020, filed on November 3, 2008.

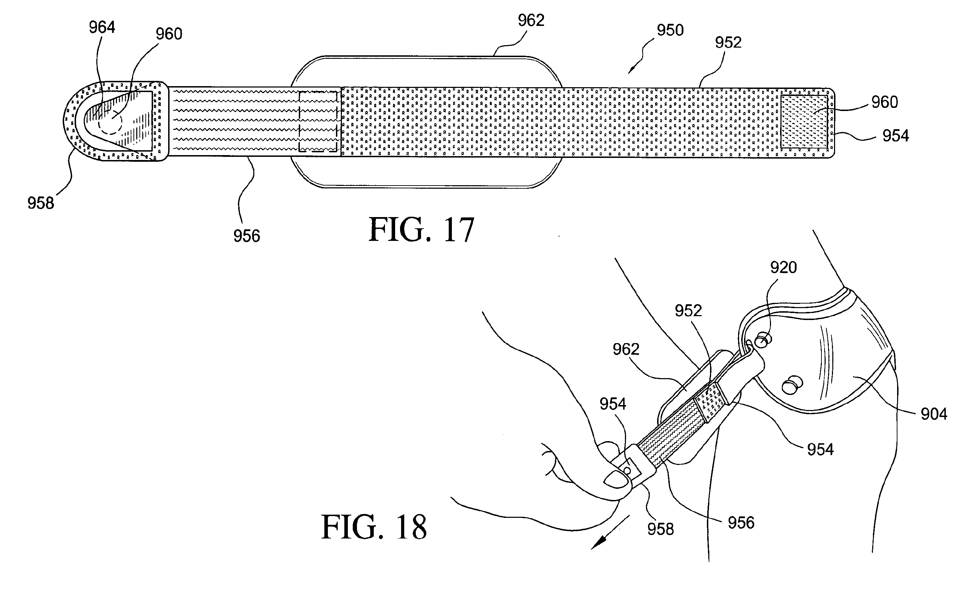

As illustrated in Fig. 17, the gastroc strap 912 is arranged to secure to the first and second sides of the lower frame element. The gastroc strap 912 has a first end defined by a strap tab 960 formed from a rigid or substantially rigid material, such as a polymer, that is arranged to secure to a first side of the lower frame member 904. A flexible handle portion 962 extends from the strap tab 958 so as to facilitate adjustment of the gastroc strap 912. A knob 964 extends from a surface of the strap tab 960 so as to secure to eyelet formed of the lower frame element, as shown in many embodiments described herein.

The gastroc strap 912 also includes a stretchable segment 956 having a first end secured to the strap tab 958, and a spanning segment 952 connected to a second end of the stretchable segment 956. The spanning segment 952 is preferably inelastic however it can be provided with some stretchability depending on various applications of the brace. The spanning segment 952 defines a second end 954 of the gastroc strap 912 and a first surface of the spanning segment 952 carries a section of hook material 960 on a first surface of the gastroc strap 912.

An unbroken loop material enabling the hook material 960 defines a second surface of the spanning segment 952. The spanning segment 952 is adjustably secured to a second side of the lower frame element such that the spanning segment 952 is adjustable in length relative to the second frame member side by securing to the slot.

As shown in Figs. 15 and 16, the gastroc strap 912 is configured for extending laterally about and over the gastroc region of the lower leg.

Fig. 18 illustrates a second side of the lower frame element 904 which defines a slot 920 whereat the second end 954 of the spanning segment 952 secures. The second end 954 wraps around the slot 920 such that the hook material 960 secures to the second surface of the spanning segment 952. The length of the gastroc strap 912 may be modified by readjusting the second end 954 relative to portions of the second surface of the spanning segment 952. By securing the second end of the gastroc strap to the lower frame element 904, the stretchable segment 958 can be tensioned over the gastroc region of the lower leg and the first end of the gastroc strap is secured to a first side of the lower frame element via the strap tab 958.

The gastroc strap includes a pad 950 arranged to adjustably and removably secure to the second surface of the spanning segment 952. The pad 950 is configured to extend over the gastroc region of the lower leg, thereby creating pressure against the gastroc region of the lower leg.

In reference to Fig. 19, the thigh strap 916 is shown as having a plurality of distinct sections having different sections. Central to the thigh strap 916 is a stretchable section 970. The stretchable section 970 has a second surface (not shown) bearing hook fastener that secures to first loop fastener surfaces of first and second end portions 978, 980 to first and second flexible sections 972, 974, respectively. The first and second flexible sections 972, 974 are preferably inelastic.

The first flexible section 972 removably secures to an extension 982 of a strap tab 976. The strap tab 976 is rigid or semi-rigid and has a curvature generally corresponding to the upper frame element 902. The strap tab 976 includes a slot 984 arranged obliquely relative to the stretchable segment 970, and is arranged for retaining a force strap. The strap tab 976 also includes a knob 986 adapted to secure to a first side of the upper frame element 902.

The stretchable section 970 secures to a first end portion of the second flexible section 972. The second flexible section 972 defines a second end portion 989 having pull sections 921, 923 arranged to extend through slots (at least one) 925, 927 formed by the upper frame element 904 so as to permit adjustment second flexible section 972 in relation to locations along the stretchable section 970.

According to one variation, the first end portion of the second flexible section 972 secures to the second surface of the stretchable section 970. The stretchable section 970 includes loop material 991 on its first surface at the second end portion 980. The second end portion 989 extends through the slot on the upper frame element 925, 927 and carries a hook material 993. The hook material 993 removably secures to the loop material 991 carried by the stretchable section 910 so as to permit adjustment of length of the thigh strap 916.

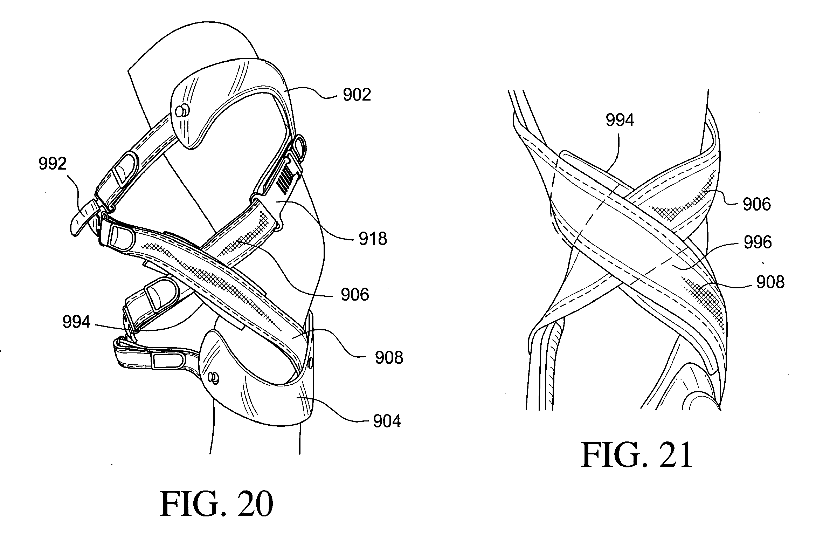

Turning to the embodiment of Figs. 20 and 21, a central strap pad 994 connects to a second surface the second strap 908. A passageway 996 is formed between the second surface of the second force strap 908 and the central strap pad 994 through which the first strap 906 extends. The first force strap 906 is movable through the passageway 996 relative to the second force strap 908.

The central strap pad 994 is removably adjustable along the second surface of the second strap. The central strap pad 994 maintains the second force strap 908 at an oblique angle relative to the first force strap 906.

The central strap pad 994 includes end portions 995, 997 extending along the second surface of the second force strap 908 and away from the passageway 996 so as to provide addition padding at the region whereat intersection of the first and second force straps 906, 908 occurs. In addition, the central strap pad 994 has a greater width relative to a width of the second force strap 908.

The configuration of the central strap pad 994 provides added comfort to the wearer, in particular at the region whereat the most amount of force is applied to the knee to relieve compartmental arthritis. Moreover, by maintaining the first and second straps together at a desired angle, the process of donning and doffing the brace is improved.

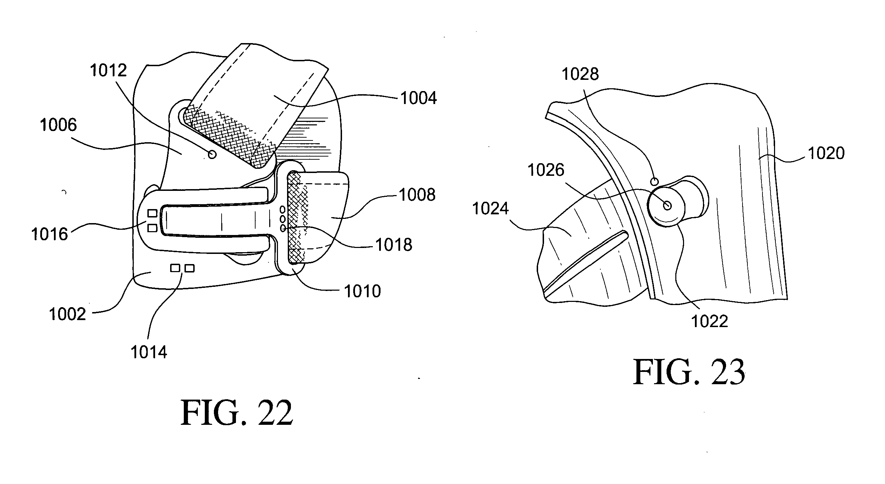

In reference to the schematic views of Figs. 22 and 23, various means are provided to assist the wearer in connecting the appropriate straps at appropriate locations on the upper and lower frame elements. For example, Fig. 22 shows a single relief dot 1012 located on a bracket carrying a first force strap 1004. The buckle assembly 1010 includes three relief dots 1018 pointing to where the lower stability strap 1008 should secure. The buckle assembly 1010 also includes two relief squares 1016 to match the relief squares 1014 located on the lower frame element 1002 so as to indicate on which frame element the buckle assembly 1010 should secure.

Fig. 23 shows how the other end of the first force strap 1004 (via a tightening mechanism) carrying a strap tab 1022 having a single relief dot 1026 secures to the upper frame element at corresponding single relief dot 1028 at an eyelet 1030.

It should be understood that the invention is not limited to relief dots or squares, but any form of indicia or color may be used to indicate whereat the various straps secure relative to the upper and lower frame elements.

The various embodiments of knee braces described above in accordance with present invention thus provide a product that reduces pain, speeds a healing process, and imparts improved stability to the knee. The knee brace is lightweight and has a streamlined profile that is simple to use for wearers of the brace of various age groups. Moreover, the knee brace permits more precise adjustment of the brace and enables efficient coordination between a medical professional and the wearer as to the degree the knee brace should be configured. Patient comfort is also enhanced and donning and doffing of the brace is eased with the novel features of the present knee brace.

Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The skilled artisan will recognize the interchangeability of various features from different embodiments. In addition to the variations described herein, other known equivalents for each feature can be mixed and matched by one of ordinary skill in this art to construct a knee brace in accordance with principles of the present invention.

The skilled artisan will also recognize that the features and concepts described herein may be extended to a variety of orthopedic applications and supports such as those employed in knee supports, ankle supports, wrist and hand supports, spinal supports, neck supports, back supports, and any other types of orthopedic supports used to assist and worn on human anatomy.

Although this invention has been disclosed in the context of certain preferred embodiments and examples, it therefore will be understood by those skilled in the art that the present invention extends beyond the specifically disclosed embodiments to other alternative embodiments and/or uses of the invention and obvious modifications and equivalents thereof. Thus, it is intended that the scope of the present invention herein disclosed should not be limited by the particular disclosed embodiments described above, but should be determined only by a fair reading of the claims that follow.

## Claims

1. A spacer element (700) configured to be used in an orthopedic or prosthetic device, the spacer element having first and second outer surfaces defined on opposed first and second sides, respectively, of the spacer element, the spacer element comprising:
a three-dimensional knit fabric layer (712) defining first and second fabric layers (91, 90) spaced by a ventilated and compressible core (94), the first fabric layer (91) being a fabric mesh having a plurality of uniformly spaced apertures (720) arranged in a pattern; and
a discrete and continuous web-like frictional layer (714) defined as a sheet of silicone elastomer laminated onto the first fabric layer (91), the frictional layer (714) having a plurality of apertures (720) arranged in a pattern, the pattern of the frictional layer apertures (720) are in direct correspondence to the pattern of the first fabric layer apertures (720);
**characterized by** a compressible material layer (716) having opposed first and second surfaces, the compressible material layer first surface secured to the second fabric layer (90),
the compressible material layer (716) having a first compression molded region (704) with reduced thickness (702, 704, 706, 708)
and increased density relative to a second region (702) of the compressible material layer (716) having a predetermined thickness.

2. The spacer element according to claim 1, wherein first compression molded area (704) retains a compression molded thickness and density.

3. The spacer element according to claim 1 or 2, wherein the first outer surface (718) is continuously flat.

4. The spacer element according to any one of claims 1 to 3, wherein the compressible material layer (716) has a plurality of different thicknesses, such that a first surface of the compressible material layer (716) is substantially flat and the second surface of the compressible material layer (716) varies in height.

5. The spacer element according to any one of claims 1 to 4, wherein the compressible layer material (716) defines at least one concave region (722) extending inwardly relative to the predetermined thickness and/or wherein the compressible material layer (716) defines at least one convex region (724) extending outwardly relative to the predetermined thickness.

6. The spacer element according to any one of claims 1 to 5, wherein a length of each of the apertures (720) is generally vertically oriented.

7. The spacer element according to any one of claims 1 to 6, further comprising a transitional region (708) extending about at least a portion of a periphery of the spacer element, the compressible material layer (716) being compressed over the transitional region (708) and the fabric layer (712) being only minimally compressed over the transitional region (708).

8. The spacer element according to any one of claims 1 to 7, further comprising a flexible region (706) wherein the second fabric layer (90) secures directly to a hook-receivable layer (718), the hook-receivable layer (718) defining the first outer surface and the frictional layer (714) defining the entirety of the second outer surface, wherein preferably the flexible region (706) is located between opposed fillet regions (726) of the spacer element, the compressible material layer (716) having a variable thickness across the fillet regions (726) and terminates at edges of the flexible region (704).

9. The spacer element according to any one of claims 1 to 8, wherein the frictional layer (714) defines the second outer surface and extends over the compressible material layer (716) such that the frictional layer (714) extends over the first and second regions (702, 708) of the compressible material layer (716).

10. The spacer element according to any one of claims 1 to 9, wherein the fabric layer (712) has a reduced thickness between a peripheral edge portion (704) lacking the compressible material layer (716) and a transitional region (708) at which the compressible material layer (716) has a variable thickness.

## Patentansprüche

1. Abstandshalterelement (700), das eingerichtet ist, in einer orthopädischen oder prothetischen Vorrichtung verwendet zu werden, wobei das Abstandshalterelement eine erste und zweite äußere Oberfläche aufweist, die auf gegenüberliegenden ersten bzw. zweiten Seiten des Abstandshalterelements definiert sind, wobei das Abstandshalterelement umfasst:
eine dreidimensionale Maschenstoffschicht (712), die eine erste und zweite Stoffschicht (91, 90) definiert, die durch einen belüfteten und komprimierbaren Kern (94) voneinander beabstandet sind, wobei die erste Stoffschicht (91) ein Stoffnetz ist, das eine Mehrzahl von gleichförmig beabstandeten Öffnungen (720) aufweist, die in einem Muster angeordnet sind, und
eine diskrete und kontinuierliche gewebeartige Reibungsschicht (714), die als ein Blatt aus einem Silikonelastomer definiert ist, das auf die erste Stoffschicht (91) laminiert ist, wobei die Reibungsschicht (714) eine Mehrzahl von Öffnungen (720) aufweist, die in einem Muster angeordnet sind, wobei das Muster der Öffnungen (720) der Reibungsschicht in direkter Entsprechung zum Muster der Öffnungen (720) der ersten Stoffschicht steht,
**gekennzeichnet durch** eine Schicht (716) aus komprimierbarem Material, die gegenüberliegende erste und zweite Oberflächen aufweist, wobei die erste Oberfläche der Schicht aus komprimierbarem Material an der zweiten Stoffschicht (90) befestigt ist, wobei die Schicht (716) aus komprimierbarem Material eine erste formgepresste Region (704) mit reduzierter Dicke (702, 704, 706, 708) und erhöhter Dichte relativ zu einer zweiter Region (702) der Schicht (716) aus komprimierbare Material mit einer vorgegebenen Dicke aufweist.

2. Abstandshalterelement nach Anspruch 1, wobei der erste formgepresste Bereich (704) eine formgepresste Dicke und Dichte behält.

3. Abstandshalterelement nach Anspruch 1 oder 2, wobei die erste äußere Oberfläche (718) kontinuierlich flach ist.

4. Abstandshalterelement nach einem der Ansprüche 1 bis 3, wobei die Schicht (716) aus komprimierbarem Material eine Mehrzahl von verschiedenen Dicken aufweist, so dass eine erste Oberfläche der Schicht (716) aus komprimierbarem Material im Wesentlichen flach ist und die zweite Oberfläche der Schicht (716) aus komprimierbarem Material in der Höhe variiert.

5. Abstandshalterelement nach einem der Ansprüche 1 bis 4, wobei die Schicht (716) aus komprimierbarem Material zumindest eine konkave Region (722) definiert, die sich nach innen relativ zu der vorgegebenen Dicke erstreckt, und/oder wobei die Schicht (716) aus komprimierbarem Material zumindest eine konvexe Region (724) definiert, die sich nach außen relativ zu der vorgegebenen Dicke erstreckt.

6. Abstandshalterelement nach einem der Ansprüche 1 bis 5, wobei eine Länge jeder Öffnung (720) im Allgemeinen vertikal orientiert ist.

7. Abstandshalterelement nach einem der Ansprüche 1 bis 6, des Weiteren umfassend eine Übergangsregion (708), die sich um zumindest einen Teil eines Umfangs des Abstandshalterelements erstreckt, wobei die Schicht (716) aus komprimierbarem Material über die Übergangsregion (708) komprimiert ist und die Stoffschicht (712) über die Übergangsregion (708) nur minimal komprimiert ist.

8. Abstandshalterelement nach einem der Ansprüche 1 bis 7, des Weiteren umfassend eine flexible Region (708), wobei die zweite Stoffschicht (90) direkt an einer Schicht (718) befestigt ist, die Haken aufnehmen kann, wobei die Schicht (718), die Haken aufnehmen kann, eine erste äußere Oberfläche definiert und die Reibungsschicht (714) die gesamte zweite äußere Oberfläche definiert, wobei sich die flexible Region (706) vorzugsweise zwischen gegenüberliegenden gerundeten Regionen (726) des Abstandshalterelements befindet, wobei die Schicht (716) aus komprimierbarem Material eine variable Dicke entlang der gerundeten Regionen (726) aufweist und an Rändern der flexiblen Region (704) endet.

9. Abstandshalterelement nach einem der Ansprüche 1 bis 8, wobei die Reibungsschicht (714) eine zweite äußere Oberfläche definiert und sich über die Schicht (716) aus komprimierbarem Material erstreckt, so dass sich die Reibungsschicht (714) über die erste und zweite Region (702, 708) der Schicht (716) aus komprimierbarem Material erstreckt.

10. Abstandshalterelement nach einem der Ansprüche 1 bis 9, wobei die Stoffschicht (712) eine reduzierte Dicke zwischen einem umlaufenden Randteil (704) ohne die Schicht (716) aus komprimierbarem Material und einer Übergangsregion (708), an welcher die Schicht (716) aus komprimierbarem Material eine variable Dicke aufweist, aufweist.

## Revendications

1. Elément d'espacement (700) configuré pour être utilisé dans un dispositif orthopédique ou prothétique, l'élément d'espacement ayant des première et seconde surfaces externes définies sur les premier et second côtés opposés, respectivement de l'élément d'espacement, l'élément d'espacement comprenant :
une couche de tissu tricoté tridimensionnelle (712) définissant des première et seconde couches de tissu (91, 90) espacées par un coeur ventilé et compressible (94), la première couche de tissu (91) étant une maille de tissu ayant une pluralité d'ouvertures uniformément espacées (720) agencées selon un modèle ; et une couche de frottement en forme de bande distincte et continue (714) définie comme étant une feuille d'élastomère silicone déposée en couche sur la première couche de tissu (91), la couche de frottement (714) ayant une pluralité d'ouvertures (720) agencées selon un modèle, le modèle des ouvertures (720) de la couche de frottement est en correspondance directe avec le modèle des premières ouvertures (720) de la couche de tissu ;
**caractérisé par** une couche de matériau compressible (716) ayant des première et seconde surfaces opposées, la première surface de la couche de matériau compressible étant fixée sur la seconde couche de tissu (90), la couche de matériau compressible (716) ayant une première région de compression moulée (704) avec une épaisseur réduite (702, 704, 706, 708) et de densité accrue par rapport à une seconde région (702) de la couche de matériau compressible (716) ayant une épaisseur prédéterminée.

2. Elément d'espacement selon la revendication 1, dans lequel la première zone de compression moulée (704) conserve une épaisseur moulée par compression et une densité.

3. Elément d'espacement selon la revendication 1 ou 2, dans lequel la première surface externe (718) est plate de manière continue.

4. Elément d'espacement selon l'une quelconque des revendications 1 à 3, dans lequel la couche de matériau compressible (716) a une pluralité d'épaisseurs différentes, de sorte qu'une première surface de la couche de matériau compressible (716) est sensiblement plate et que la seconde surface de la couche de matériau compressible (716) varie en hauteur.

5. Elément d'espacement selon l'une quelconque des revendications 1 à 4, dans lequel le matériau de couche compressible (716) définit au moins une région concave (722) s'étendant vers l'intérieur par rapport à l'épaisseur prédéterminée et/ou dans lequel la couche de matériau compressible (716) définit au moins une région convexe (724) s'étendant vers l'extérieur par rapport à l'épaisseur prédéterminée.

6. Elément d'espacement selon l'une quelconque des revendications 1 à 5, dans lequel une longueur de chacune des ouvertures (720) est généralement orientée verticalement.

7. Elément d'espacement selon l'une quelconque des revendications 1 à 6, comprenant en outre une région de transition (708) s'étendant autour d'au moins une partie d'une périphérie de l'élément d'espacement, la couche de matériau compressible (716) étant comprimée sur la région de transition (708) et la couche de tissu (712) étant uniquement minimalement comprimée sur la région de transition (708).

8. Elément d'espacement selon l'une quelconque des revendications 1 à 7, comprenant en outre une région flexible (706) dans laquelle la seconde couche de tissu (90) se fixe directement sur une couche de réception de crochet (718), la couche de réception de crochet (718) définissant la première surface externe et la couche de frottement (714) définissant l'intégralité de la seconde surface externe, dans lequel de préférence la région flexible (706) est positionnée entre des régions de bande (726) opposées de l'élément d'espacement, la couche de matériau compressible (716) ayant une épaisseur variable sur les régions de bande (726) et se termine au niveau des bords de la région flexible (704).

9. Elément d'espacement selon l'une quelconque des revendications 1 à 8, dans lequel la couche de frottement (714) définit la seconde surface externe et s'étend sur la couche de matériau compressible (716) de sorte que la couche de frottement (714) s'étend sur les première et seconde régions (702, 708) de la couche de matériau compressible (716).

10. Elément d'espacement selon l'une quelconque des revendications 1 à 9, dans lequel la couche de tissu (712) a une épaisseur réduite entre une partie de bord périphérique (704) dépourvue de couche de matériau compressible (716) et une région de transition (708) au niveau de laquelle la couche de matériau compressible (716) a une épaisseur variable.
